# EUROPEAN PATENT APPLICATION

(11) **EP 1 707 227 A2**
(43) Date of publication of application: **04.10.2006**
(21) Application number: 06251837.8
(22) Date of filing: 31.03.2006
(51) Int. Cl.: A61M 5/142, A61B 17/80

(54) **Plate for fusion of the metatarso-phalangeal joint**

(30) Priority: 31.03.2005 US 94972
(71) Applicant: DePuy Products, Inc., Warsaw, IN 46581 (US)
(72) Inventor: Sanders, Roy, Tampa, FL 33606 (US); Bremer, Chris, Warsaw, IN 46582 (US); Prasad, Priya, Warsaw, IN 46580 (US)
(74) Representative: Belcher, Simon James

(57) **Abstract**

A fixation plate for use in fusion of the metatarsal-phalangeal joint includes a distal portion configured to engage the metatarsus bone and a proximal portion configured to engage the phalanx bone. The distal portion is elongated with several screw defined through it along a longitudinal axis passing through the portion. The proximal portion includes a plurality of screw holes that are all offset relative to each other along axes parallel and perpendicular to the longitudinal axis. No more than one of the screw holes in the proximal portion is aligned with the longitudinal axis. The fixation plate is contoured to cup the bones of the MTP joint. The plate may include an intermediate portion that is bent at a pre-determined dorsi-flexion angle.

## Description

The present invention relates to the general technical field of surgical devices for fixing together and aligning the two bony parts of a joint relative to each other, and in particular a metatarso-phalangeal joint, in order to perform arthrodesis.

Arthrodesis or fusion of the first metatarso-phalangeal (MTP) joint is often the treatment of choice for several indications, such as hallux valgus, hallux limitus or rigidus, degenerative joint disease, severe dislocation or subluxation, and degenerative deformities. Fusion can be used to correct deformities associated with these indications or to alleviate joint pain associated with movement of the MTP joint. Fusion of the first MTP joint allows the patient to walk without discomfort, usually with minimal impact on gait pattern. In fact, where the indicated pathology led to a significant disruption in the patient's ability to walk, fusion may actually improve the patient's gait pattern.

As a general rule, arthrodesis can be problematic because it results in a joint position that is defined and irreversible. Thus, it is very important for arthrodesis of an MTP joint to be performed carefully so that the two bones will be accurately positioned relative to each other to avoid any subsequent difficulty and to preserve the patient's ability to walk as normally as possible.

In one common fusion procedure, the articulating aspects of the metatarsal bone and phalanx are prepared as necessary so that the bones can be positioned at appropriate dorsi-flexion and varus-valgus angles. Then a pair of bone fasteners, such as 4-0 cannulated screws, are implanted across the MTP joint to fix the joint position. Bone graft may be introduced in areas of bone separation to facilitate complete fusion of the joint.

In one alternative, a fixation plate is implanted across the joint and is typically fastened to the opposing bones by bone screws. The fixation plate is bent by the surgeon to achieve an angle in the dorsi-flexion plane that is specific to the patient, thereby reducing difficulty for the patient while walking and minimizing possible future complications. Many prior plates are unsuitable for bending through a varus-valgus angle, which means that they are not capable of implementing arthrodesis that is sufficiently close to the optimum anatomic orientation of the two bones to be fused together.

More recently, fixation plates have been provided that are pre-formed with a fixed varus-valgus angle and a fixed dorsi-flexion angle. An example of this type of plate is the plate which is available from Newdeal S A under the trade mark HALLU-C. This plate constitutes two linear plate sections aligned at a fixed 10° varus-valgus angle relative to each other. The plate is also bent at its mid-line to form a 10° dorsi-flexion angle. Each linear plate section includes an elongated slot flanked by two screw holes arranged along the longitudinal axis of the section. Other details of this plate appear in US-A-2003/ 0060827.

Fixation plates of this type represent an improvement over prior plates that required the surgeon to bend the plate at the dorsi-flexion angle during the surgical procedure and that do not permit any varus-valgus angle. However, there is still room for improvement in fixation plates for the MTP joint, especially for the first MTP joint. In particular, there is a need for a fixation plate that provides greater flexibility in positioning the bone fasteners fixing the plate to the associated bones, especially the phalanx. There is also a need for a fixation plate that presents a lower profile to minimize soft tissue irritation.

The present invention satisfies the need for an improved fixation plate for arthrodesis of the metatarsal-phalangeal joint. In one embodiment of the invention, the plate includes a metatarsal portion that is generally elongated to extend along the distal length from the head of the metatarsus bone. A series of chamfered screw holes extend along the axis of the metatarsal portion.

The plate further includes a phalanx portion connected to the metatarsal portion by an intermediate portion of the plate. The phalanx portion is enlarged and asymmetric relative to the elongated metatarsal portion. In the preferred embodiment, the phalanx portion includes a medial wing and an opposite lateral wing that is axially offset from the medial wing. Each wing supports a chamfered screw hole. A central region between the two wings also supports thee screw holes, laterally and axially offset from each other.

In a further aspect of the invention, the entire plate is curved to provide a curved bone engaging surface that generally follows the contour of the metatarsal bone and phalanx. Rather than incorporate a pre-determined varus-valgus angle offset between the metatarsal portion and phalanx portion of the plate, the fixation plate of the present invention is configured so that the phalanx portion cups the proximal end or base of the phalanx and the metatarsal portion simply overlays the distal portion of the metatarsus bone to be fixed to the bone at whatever angle is dictated by the orientation of the phalanx portion fixed to the base of the phalanx. In the preferred embodiment, the plate is bent at the intermediate portion to an appropriate dorsi-flexion angle.

In order to minimize the profile of the plate, the present invention contemplates a plate thickness of about 1 mm. In addition, the perimeter of the plate is contoured about the screw holes to reduce the amount of material across the surface of the plate.

One benefit of the present invention is that it provides a plate for arthrodesis of the MTP joint that can be firmly fixed to the bones of the joint. Another benefit is that the plate provides for a variety of screw fixation points, especially across the base of the phalanx.

A further benefit achieved by the fixation plate of the present invention is that is exhibits a minimal profile to reduce its prominence over the bones and minimizes tissue irritation.

Embodiments of the invention will now be described by way of example with reference to the accompanying drawings, in which:
FIG. 1 is a top view of the metatarsal-phalangeal joint with a fixation plate situated thereon in accordance with one embodiment of the present invention.
FIG. 2 is a top flat pattern view of the fixation plate shown in FIG. 1.
FIG. 3 is a bottom view of the fixation plate illustrated in FIG. 1, especially showing the curvature of the bone engaging surface of the plate.
FIG. 4 is a longitudinal cross sectional view of the plate shown in FIG. 3, taken along line 4-4 as viewed in the direction of the arrows.
FIG. 5 is an end view of the plate depicted in FIG. 3.

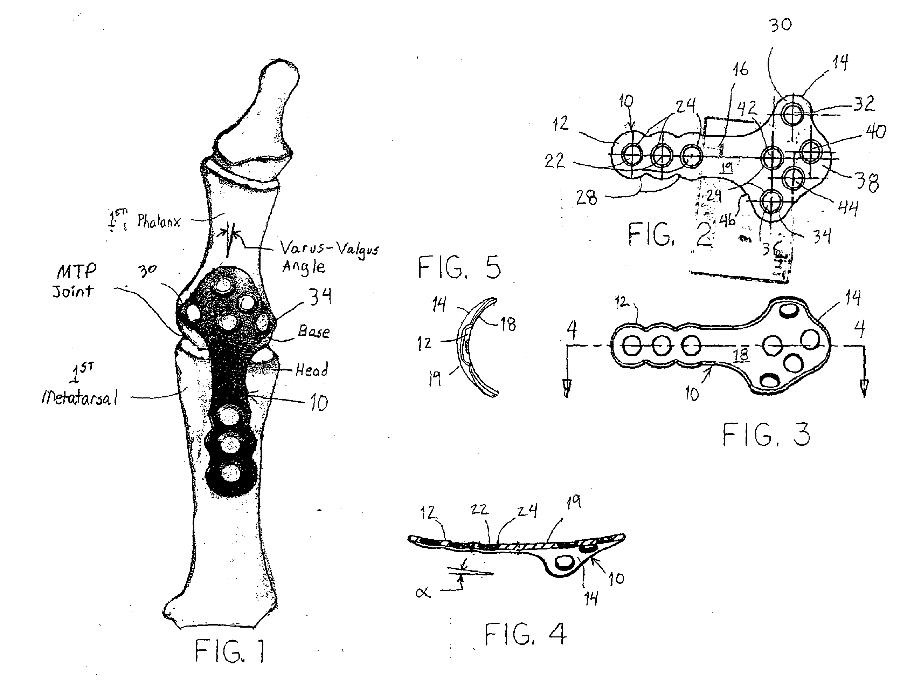

Referring to the drawings, the distal bones of the first toe, or great toe, are shown in FIG. 1. In particular, the toe includes a first metatarsal bone, a first phalanx bone and a metatarsal-phalangeal (MTP) joint between them. A fixation plate 10 according to one embodiment of the present invention spans the MTP joint and is configured to be fixed to both bones of the joint. As shown in more detail in FIG. 2, the plate 10 includes a distal or metatarsal portion 12 and a proximal or phalanx portion 14. An integral intermediate portion 16 connects the distal and proximal portions.

The distal portion 12 is generally elongated with at least two, and most preferably three, holes 22 positioned substantially along the longitudinal axis of the portion. The holes 22 are configured to receive the shank of a bone fastener, such as a bone screw. In one aspect of this embodiment, the holes 22 include a circumferential chamfer 24. This chamfer allows the use of two different sizes of bone screw. In a specific embodiment, the holes 22 have a diameter of 3.8 mm with a 120° chamfer 24 to produce a proximal diameter of 5.3 mm. This specific screw hole is configured to receive either 2.7 mm or 3.5 mm cortical screws.

In the illustrated embodiment, the screw holes 22 are configured to receive non-locking screws. In an alternative embodiment, the screw holes are designed to receive locking screws, such as by the incorporation of locking threads (not shown) within the screw hole. The locking threads can be of a variety of known configurations as dictated by the particular cortical locking screw. In a specific embodiment, the locking threads may be at 0.5 mm pitch, with a 4.0 mm major diameter and a 3.6 mm minor diameter.

In the preferred embodiment of the invention, the screw holes 22 are spaced at 6.0 mm intervals. In one aspect of the invention, the perimeter 28 of the distal portion 12 is contoured around the screw holes to reduce the plate material in the area around the holes that does not carry any appreciable load.

The proximal or phalanx portion 14 of the plate is asymmetric, as best seen in FIG. 2. In the preferred embodiment, the proximal portion includes a medial wing 30 and an opposite lateral wing 34. The two wings are axially offset from each other, with the medial wing being positioned more proximal than the lateral wing. The configuration of the wings 30, 34 generally correspond to the orientation of the base of the first phalanx when the phalanx is positioned at an acceptable varus-valgus angle, as shown in FIG. 1. An acceptable varus-valgus angle may range from 5 to 10°, with 5° being most preferred for the majority of patients. At this angle, the medial aspect of the base of the phalanx is more proximal than the lateral aspect. This offset is accounted for in the plate 10 by the axial offset between the medial and lateral wings.

Each wing supports a corresponding screw hole 32, 36. The screw holes are preferably configured like the screw holes 22 described above to include the circumferential chamfer 24. The screw holes 32, 36 may accept locking or non-locking cortical screws, as described above. As with the screw holes in the distal portion 12, the perimeter of each wings 30, 34 is contoured around the corresponding screw holes 32, 36, to reduce the plate profile or prominence above the bone and minimize soft tissue irritation.

The proximal portion 14 includes a central region 38 between the two wings. In the preferred embodiment, the central region 38 includes three screw holes 40, 42 and 44. The centre of each of these screw holes is axially and transversely offset relative to each other, as well as relative to the screw holes 32, 36 in the wings. The screw hole 42 may be axially aligned with the screw holes 22 in the distal portion 12 of the plate 10 and/or along the longitudinal axis of the distal portion.

In the preferred embodiment, the proximal portion 14 of the plate includes five screw holes 32, 36, 40, 42 and 44. The screw holes are arranged so that a screw can be threaded into the phalanx through each hole without conflict. The surgeon may select all or any subset of the screw holes for fixation of the plate 10 to the phalanx. This flexibility in screw placement is particularly beneficial for patients with osteopenic bone, where a portion of the bone has been resected or in cases where the base of the phalanx has been fractured. Moreover, the arrangement of the screw holes across the proximal portion 14 allows the surgeon to select a minimum of two screw positions that optimally affixes the plate 10 to the bone. In other words, with the five screw holes in the proximal portion, the surgeon can introduce two bone screws in ten different orientations (e.g., placing a screw in holes 32 and 36, or in holes 40 and 36, or in holes 42 and 44).

It can be appreciated that the arrangement of screw holes in the plate 10 accommodates any varus-valgus angle at the time of implantation. In one method for implanting the plate 10, the surgeon determines the number of screws necessary for strong attachment of the plate to the phalanx. The proximal portion 14 of the plate is positioned on the phalanx and the bone screws are driven into the bone to attach the plate to the bone. (It is understood that the bone is prepared to receive the bone screws according to accepted practice, such as by pre-drilling and tapping a bore in the bone). With the proximal portion attached to the phalanx, the phalanx can be positioned at an acceptable varus-valgus angle. In so doing, the distal portion 12 of the plate 10 will shift position relative to the metatarsal bone, but will always maintain sufficient contact with the bone.

Once the acceptable phalanx-metatarsal bone angle has been achieved, the distal portion 12 may then be attached to the metatarsal bone using any combination of bone screws in the screw holes 22. It can therefore be appreciated that the fixation plate 10 of the present invention eliminates the difficulty associated with prior fusion plates in achieving or accommodating an acceptable varus-valgus angle. The plate 10 accepts any angle desired by the surgeon and does not enforce a pre-determined varus-valgus angle like prior plates. With the screw hole arrangements of the plate 10 of the present invention, the surgeon can produce valgus angles ranging from about 5° to about 10° for fixation of the first toe.

This aspect of the plate 10 also facilitates manufacture of the plate. In one manner of making the plate, a sheet of material may be stamped into the flat pattern shape shown in FIG. 2. The necessary edge and surface treatments (such as deburring and anodizing) are easily accomplished on the flat pattern. The flat pattern may then be bent over a mandrel to introduce the curvature of the bone engaging surface 18, as well as the upper surface 19 curvature, as both described below.

In a further feature of the fixation plate 10, the bone engaging surface 18 of the plate may be contoured at a radius approximating the surface of the bone, as shown in FIGS. 3 and 5. In a preferred embodiment, both the entire plate 10 is contoured along its length at a radius of about 9.3 mm. In other embodiments, only the proximal portion 14 is contoured to fit the base of the phalanx. In addition to reducing the prominence of the fixation plate 10 above the bone, the contoured surface 18 also acts to "cup" the bone, especially the base of the phalanx. This "cupping" feature enhances the fixation of the plate to the bone, helps reduce fractures in the phalanx and helps align the bone screw axis to produce maximum engagement within the bone.

In addition to the contour of the surface 18, the plate may also incorporate a curvature of the upper surface 19 along the length of the plate, as best seen in FIG. 4. This gradual curvature helps maintain a solid contact between the plate 10 and the two bones of the joint. In a specific embodiment, the plate is curved at a radius of about 130 mm over the length of the plate.

The fixation plate 10 preferably incorporates a pre-determined dorsi-flexion angle α, as shown in FIG. 4. In particular, the plate is bent at the intermediate portion 16 so that the bend can be oriented at the MTP joint between the two bones. In a most preferred embodiment, the plate is pre-bent at a dorsi-flexion angle of about 17° for the first toe, which has been found to be anatomically optimal for most patients. However, the plate 10 can be offered pre-bent at other dorsi-flexion angles, and may even be bent by the surgeon to a different angle.

The plate 10 is formed of any medical grade material that is sufficiently strong to support the toe until fusion is achieved. In the preferred embodiment, the plate is formed of a titanium alloy, such as Ti-6A1-4V. In order to maintain a minimal profile, the plate has a thickness of about 1 mm.

In the illustrated embodiment, the plate 10 is configured for the right foot of the patient. It is of course understood that a plate for the left foot will assume a mirror image of the plate shown in FIGS. 1 to 3.

## Claims

1. A fixation plate for fusion of the metatarsal-phalangeal (MTP) joint between a metatarsal bone and a phalanx bone, said plate comprising:
an elongated distal portion configured for attachment to the metatarsal bone, said distal portion defining at least one distal opening along a longitudinal axis passing through said distal portion, said at least one distal opening configured to receive a bone engaging fastener therethrough; and
a proximal portion connected to said distal portion and defining a plurality of proximal openings configured to receive a bone engaging fastener therethrough, wherein no more than one of said plurality of openings is aligned with said longitudinal axis.

2. The fixation plate according to claim 1, wherein said proximal portion includes a pair of opposite wings laterally offset from said longitudinal axis, each of said wings including one of said plurality of proximal openings.

3. The fixation plate according to claim 2, wherein said proximal portion further includes a central region between said wings, said central region defining at least one of said plurality of proximal openings.

4. The fixation plate according to claim 3, wherein said central region defines three of said plurality of proximal openings.

5. The fixation plate according to claim 4, wherein all of said proximal openings are offset relative to each other along an axis parallel to said longitudinal axis.

6. The fixation plate according to claim 5, wherein all of said proximal openings are offset relative to each other along an axis perpendicular to said longitudinal axis

7. The fixation plate according to claim 1, wherein at least said proximal portion exhibits a curvature in a surface of said proximal portion contacting the phalanx bone adapted to generally conform to the surface of the bone.

8. The fixation plate according to claim 1, wherein said plate exhibits a curvature along said longitudinal axis away from the MTP joint.

9. The fixation plate according to claim 1, wherein at least some of said distal and proximal openings include a circumferential chamfer at the surface of said plate opposite the MTP joint.

10. The fixation plate according to claim 1, further comprising an intermediate portion connecting said proximal portion to said distal portion, wherein said intermediate portion does not include any openings for receiving a bone engaging fastener through it.

11. The fixation plate according to claim 10, wherein said plate is bent at said intermediate portion at a pre-determined dorsi-flexion angle.
